# EUROPEAN PATENT SPECIFICATION

(11) **EP 1 731 140 B1**
(45) Date of publication and mention of the grant of the patent: **13.04.2011**
(21) Application number: 06016853.1
(22) Date of filing: 17.09.1999
(51) Int. Cl.: A61K 9/12

(54) **Medicinal aerosol formulation**
Medizinische Aerosolformulierung
Formulation d'aerosol medicinal

(30) Priority: 22.09.1998 US 158369
(43) Date of publication of application: 13.12.2006
(62) Divisional of application: 99946974.5
(73) Proprietor: Aeropharm Technology Incorporated, Edison, NJ 08818 (US)
(72) Inventor: Adjei, Akwete, Bridgewater NJ 08807 (US); Cutie, Anthony J., Bridgewater NJ 08807 (US)
(74) Representative: Forstmeyer, Dietmar

(56) References cited:
- WO-A-90/09781
- WO-A-96/19198
- WO-A-96/32096
- US-A- 5 569 450

## Description

### BACKGROUND OF THE INVENTION

### Field of the Invention

This invention relates to a medicinal aerosol formulation, and more particularly, to a medicinal aerosol formulation comprising a stabilizer selected from an amino acid, a derivative thereof or a mixture of the foregoing.

### Description of the Related Art

Delivery of drugs to the lung by way of inhalation is an important means of treating a variety of conditions, including such common local conditions as bronchial asthma and chronic obstructive pulmonary disease and some systemic conditions including pain management, cystic fibrosis, etc. Steroids, β2 agonists, anti-cholinergic agents, proteins and polypeptides are among the drugs that are administered to the lung for such purposes. Such drugs are commonly administered to the lung in the form of an aerosol of particles of respirable size (less than about 10 µm in diameter). In order to assure proper particle size in the aerosol, particles can be prepared in respirable size and then incorporated into a suspension formulation containing a propellant. Alternatively, formulations can be prepared in solution form in order to avoid the concern for proper particle size in the formulation. Solution formulations must nevertheless be dispensed in a manner that produces particles or droplets of respirable size.

Once prepared an aerosol formulation is filled into an aerosol canister equipped with a metered dose valve. In the hands of the patient the formulation is dispensed via an actuator adapted to direct the dose from the valve to the patient.

WO 90/09781 discloses a self-propelled therapeutic aerosol suspension for inhalation comprising a micronized, water soluble, propellant insoluble, homogeneous complex of a drug and an extender suspended in a propellant mixture.

US 5,569,450 discloses a medicinal aerosol formulation containing a particulate drug and a dispersing aid derived from a hydroxyacid, a mercapto acid, or an amino acid.

WO 96/19198 discloses aerosol formulations suitable for use in pressurized metered dose inhalers which comprise a hydrofluoroalkane propellant, a medicament for inhalation and a surfactant which is a C₈-C₁₆ fatty acid or salt thereof, a bile salt, a phospholipid or an alkyl saccharide.

It is important that an aerosol formulation be stable such that the pressurized dose discharged from the metered dose valve is reproducible. Rapid creaming, settling, or flocculation after agitation are common sources of dose irreproducibility in suspension formulations. This is especially true where a binary aerosol formulation containing only medicament and propellant, e.g. 1,1,1,2-tetrafluoroethane, is employed or where such formulation contains small amounts of surfactant as well. Sticking of the valve also can cause dose irreproducibility. In order to overcome these problems aerosol formulations often contain surfactants, which serve as suspending aids to stabilize the suspension for a time sufficient to allow for reproducible dosing. Certain surfactants also function as lubricants to lubricate the valve to assure smooth actuation. Myriad materials are known and disclosed for use as dispersing aids in aerosol formulations. Suitability of materials, however, is dependent on the particular drug and the propellant or class of propellant used in the formulation.

It is sometimes difficult to dissolve sufficient quantities of conventional surfactants in hydrofluorocarbon (HFC) propellants such as HFC-134a and HFC-227. Cosolvents, such as ethanol, have been used to overcome this problem, as described in U.S. Patent NO. 5,225,183. An alternative approach that avoids cosolvents involves materials that are soluble in hydrofluorocarbon propellants and are said to be effective surfactants or dispersing aids in an aerosol formulation. Among such materials are certain fluorinated surfactants and certain polyethyoxysurfactants.

### SUMMARY OF THE INVENTION

It has surprisingly been found that novel medicinal aerosol formulations can be obtained without the use of either cosolvents, such as ethanol, or surfactants, such as sorbitan trioleate which are added to a binary aerosol formulation. Stable medicinal aerosol formulations arc obtained by the use of amino acids, derivatives thereof or a mixture of the foregoing.

### DETAILED DESCRIPTION OF THE INVENTION

This invention involves a stable suspension aerosol formulation as defined in claim 1 suitable for pressurized delivery which comprises (1) a particulate medicament or drug, (2) a suitable propellant, and (3) a suitable stabilizer.

A suitable medicament or drug is one which is suitable for administration by inhalation, the inhalation being used for oral and nasal inhalation therapy. Therapeutic categories of drugs or medicaments include cardiovascular drugs, antiallergics, analgesics, brochodilators, antihistamines, antitussives, antifungals, antivirals, antibiotics, pain medicaments, antiinflammatories, peptides, proteins and steroids.

Particularly suitable medicaments or drugs include albuterol (also known as salbutamol), atropine, beclomethasone, esters of beclomethasone such as its monopropionate and dipropionate, budesonide, cromolyn, epinephrine, ephedrine, fentanyl, flunisolide, formoterol, ipratropium bromide, isoproterenol, pirbuterol, prednisolone, salmeterol, amiloride, fluticasone esters, such as phosphate, monohydrate and furoate, (-)4-amino-3,5-dichloro-α-[[[6(2-pyridinyl)ethoxy] hexyl] amino]methyl]benzene-methanol. Also included are the suitable acid addition salts of the foregoing drugs, their hydrates and their other solvates. In this regard, suitable acid addition salts include the salts obtained from inorganic acids, such as hydrochloric, hydrobromic, sulfuric, nitric, phosphoric and perchloric acids as well as organic acids such as tartaric, citric, acetic, succinic, maleic, fumaric and oxalic acids. Suitable pharmaceutically acceptable solvates include solvates with ethylactate, alkanes, ethers, alcohols and water.

For purposes of the formulations of this invention, which are intended for inhalation into the lungs, the medicament or drug is preferably micronized whereby a therapeutically effective amount or fraction (e.g., ninety percent or more) of the drug is particulate. Typically, the particles have a diameter of less than about 10 microns, and preferably less than about 5 microns, in order that the particles can be inhaled into the respiratory tract and/or lungs.

The particulate medicament or drug is present in the inventive formulations in a therapeutically effective amount, that is, an amount such that the drug can be administered as an aerosol, such as topically, or via oral or nasal inhalation, and cause its desired therapeutic effect, typically preferred with one dose, or through several doses. The particulate drug is administered as an aerosol from a conventional valve, e.g., a metered dose valve.

The term "amount" as used herein refers to quantity or to concentration as appropriate to the context. The amount of a drug that constitutes a therapeutically effective amount varies according to factors such as the potency of the particular drug, the route of administration of the formulation, and the mechanical system used to administer the formulation. A therapeutically effective amount of a particular drug can be selected by those of ordinary skill in the art with due consideration of such factors. Generally a therapeutically effective amount will be from about 0.005 parts by weight to about 2 parts by weight based on 100 parts by weight of the propellant.

A suitable propellant is selected. A suitable propellant is any fluorocarbon, e.g. a 1-4 hydrogen containing flurocarbon(,such as CHF₂CHF₂, CF₃CH₂F, CH₂F₂CH₃ and CF₃CHFCF₃₎), a perfluorocarbon, e.g. a 1-4 carbon perfluorocarbon, (such as CF₃CF₃, CF₃CF₂CF₃); or any mixture of the foregoing, having a sufficient vapor pressure to render them effective as propellants. Some typical suitable propellants include conventional chlorofluorocarbon (CFC) propellants such as mixtures of propellants 11, 12 and 114. The propellants according to the present invention are Non-CFC propellants namely 1,1,1,2-tetrafluoroethane (Propellant 134a), 1,1,1,2,3,3,3-heptafluoropropane (Propellant 227) or mixtures thereof. The propellant is preferably present in an amount sufficient to propel a plurality of the selected doses of drug from an aerosol canister.

A suitable stabilizer is selected. A suitable stabilizer includes (1) an amino acid selected from (a) a monoamino carboxylic acid of the formula, H₂N-R-COOH (I), (b) a monoamino dicarboxylic acid of the formula, H₂N-R(COOH)₂ (II) and (c) a diamino monocarboxylic acid of the formula (H₂N)₂-R COOH (III), where R is a straight or branched alkyl radical of from 1 to 22 carbon atoms, which can be mono or poly-substituted with moieties such as sulfide (-S-), oxide (-O-), hydroxyl (-OH), amide (-NH), sulfate (-SO4); aryl of the formula 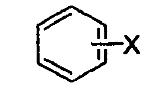 , where X is hydrogen, halogen (F, Cl, BR, I), alkyl of 1 to 6 carbon atoms, alkoxy of 1 to 6 carbon atoms, hydroxy and nitro; and heterocyclic, such as thienyl, furyl, pyranyl, imidazolyl, pyrrolyl, thizolyl, oxazolyl, pyridyl, and pyrimidinyl compounds; (2) (a) acid addition salts of the amino group, obtained from inorganic acids, such as hydrochloric, hydrobromic, sulfuric, nitric, phosphoric, and perchloric acids, as well as organic acids, such as tartaric, citric, acetic, succinic, maleic, fumaric, oxalic acids; (b) amides of the carboxylic acid group, e.g., glutamine, and (3) a mixture of the foregoing.

Suitable amino acids of the invention are of the formula I glycine, alanine, valine, leucine, isoleucine, methionine, threonine, isovaline, phenylalanine, tyrosine, serine, cysteine, histidine, tryptophan, proline, and hydroxyproline, e.g. trans-4-hydroxy proline. Compounds of the formula II are aspartic acid, and glutamic acid, compounds of the formula (III) are arginine, lysine, hydroxylysine, ornithine, asparagine, and citrulline.

An aerosol formulation preferably comprises the stabilizer in an amount effective to stabilize the formulation relative to an identical formulation not containing the stabilizer, such that the drug does not settle, cream or flocculate after agitation so quickly as to prevent reproducible dosing of the drug. Reproducible dosing can be achieved if the formulation retains a substantially uniform drug concentration for about two or three seconds after agitation.

The particular amount of stabilizer that constitutes an effective amount is dependent upon the particular stabilizer, the particular propellant, and on the particular drug used in the formulation. It is therefore not practical to enumerate specific effective amounts for use with specific formulations of the invention, but such amounts can readily be determined by those skilled in the art with due consideration of the factors set forth above. Generally, however, the stabilizer can be present in a formulation in an amount from about 0.000002 percent by weight, to about 20% by weight, more preferably about 0.0002 percent to about 10% by weight, based on the weight of the formulation.

It has surprisingly been found that the formulation of the invention is stable without the necessity of employing a cosolvent, such as ethanol, or surfactants. However, further components, such as conventional lubricants or surfactants, cosolvents, ethanol, etc., can also be present in an aerosol formulation of the invention in suitable amounts readily determined by those skilled in the art. In this regard, reference is made to U.S. Patent No. 5,225,183.

Generally the formulations of the invention can be prepared by combining (i) the drug in an amount sufficient to provide a plurality of therapeutically effective doses; (ii) the stabilizer in an amount effective to stabilize each of the formulations; (iii) the propellant in an amount sufficient to propel a plurality of doses from an aerosol canister; and (iv) any further optional components e.g. ethanol as a cosolvent; and dispersing the components. The components can be dispersed using a conventional mixer or homogenizer, by shaking, or by ultrasonic energy. Bulk formulation can be transferred to smaller individual aerosol vials by using valve to valve transfer methods, pressure filling or by using conventional cold-fill methods. It is not required that a stabilizer used in a suspension aerosol formulation be soluble in the propellant. Those that are not sufficiently soluble can be coated onto the drug particles in an appropriate amount and the coated particles can then be incorporated in a formulation as described above.

Aerosol canisters equipped with conventional valves, preferably metered dose valves, can be used to deliver the formulations of the invention. It has been found, however, that selection of appropriate valve assemblies for use with aerosol formulations is dependent upon the particular stabilizer and other adjuvants used (if any), on the propellant, and on the particular drug being used. Conventional neoprene and buna valve rubbers used in metered dose valves for delivering conventional CFC formulations often have less than optimal valve delivery characteristics and ease of operation when used with formulations containing HFC-134a or HFC-227. Therefore certain formulations of the invention are preferably dispensed via a valve assembly wherein the diaphragm is made of a nitrile rubber such as DB-218 (American Gasket and Rubber, Schiller Park, Ill.) or an EPDM rubber such as Vistalon^{™} (Exxon), Royalene^{™} (UniRoyal), bunaEP (Bayer). Also suitable are diaphragms fashioned by extrusion, injection molding or compression molding from a thermoplastic elastomeric material such as FLEXOMER^{™} GERS 1085 NT polyolefin (Union Carbide).

Conventional aerosol canisters, coated or uncoated, anodized or unanodized, e.g., those of aluminum, glass, stainless steel, polyethylene terephthalate, and coated canisters or cans with epon, epoxy, etc., can be used to contain a formulation of the invention.

The formulation of the invention can be delivered to the respiratory tract and/or lung by oral inhalation in order to effect bronchodilation or in order to treat a condition susceptible of treatment by inhalation, e.g., asthma, chronic obstructive pulmonary disease. The formulations of the invention can also be delivered by nasal inhalation in order to treat, e.g., allergic rhinitis, rhinitis, (local) or diabetes (systemic), or they can be delivered via topical (e.g., buccal) administration in order to treat, e.g., angina or local infection.

## Claims

1. A medicinal aerosol formulation, which comprises:
(a) a therapeutically effective amount of a particulate medicament;
(b) a non-CFC propellant wherein said propellant is selected from the group consisting of 1,1,1,2-tetrafluoroethane, 1,1,1,2,3,3,3-heptafluoropropane or a mixture thereof; and
(c) a stabilizer selected from an amino acid, or a mixture of the foregoing, wherein said stabilizer is selected from the group consisting of glycine, alanine, valine, leucine, isoleucine, methionine, threonine, isovaline, phenylalanine, tyrosine, serine, histidine, tryptophan, proline, hydroxyproline, arginine, ornithine, asparagine, citrulline, aspartic acid, cysteine, glutamic acid, glutamine, lysine, hydroxylysine, trans-4-hydroxy-L-proline, and a mixture of any of the foregoing.

2. The formulation as defined in claim 1 wherein the medicament is selected from the group consisting of albuterol, atropine, beclomethasone, beclomethasone monopropionate, beclomethasone dipropionate, budesonide, cromolyn, epinephrine, ephedrine, fentanyl, flunisolide, formoterol, ipratropium bromide, isoproterenol, pirbuterol, prednisone, salmeterol, amiloride, fluticasone, fluticasone esters, (-)4-amino-3,5-dichloro-a-[[[6(2-pyridinyl)ethoxy] hexyl] amino] methyl]benzene-methanol and pharmaceutically acceptable salts, esters, hydrates and solvates of the foregoing.

3. The formulation as defined in claim 1 which further includes a cosolvent.

4. The formulation as defined in claim 3 wherein said cosolvent comprises ethanol.

5. The formulation as defined in claim 1 wherein said stabilizer is present in an amount effective to prevent settling, creaming or flocculation of the formulation for a time sufficient to allow reproducible dosing of the drug after agitation of the formulation.

6. The formulation as defined in claim 5 wherein said stabilizer is present in an amount ranging from about 0.000002% by weight to about 20% by weight based on the weight of the formulation.

7. A method of preparing a medicinal aerosol formulation according to claim 1, which comprises:
(a) combining (i) said medicament in an amount sufficient to provide a plurality of therapeutically effective doses, (ii) said propellant in an amount sufficient to propel a plurality of said therapeutically effective doses from an aerosol canister; and (iii) said stabilizer in an amount effective to stabilize the formulation; and
(b) dispersing components (i), (ii) and (iii).

8. The method as defined in claim 7 wherein the medicinal aerosol formulation further comprises combining in step (a) a cosolvent and in step (b) dispersing components (i), (ii), (iii) with said cosolvent.

9. Use of a composition according to claim 1 for the reparation of a medicinal aerosol formulation for treating asthma, chronic obstructive pulmonary disease, allergic rhinitis, rhinitis, diabetes, angina or local infection.

10. A formulation according to claim 1 in an aerosol canister equipped with a metered dose valve.

11. A method of stabilizing a suspension aerosol formulation comprising a non-CFC propellant wherein said propellant is selected from the group consisting of 1,1,1,2-tetrafluoroethane, 1,1,1,2,3,3,3-heptafluoropropane or a mixture thereof and a particulate drug which comprises,
incorporating into the formulation a stabilizer selected from the group consisting of a suitable amino acid, or any mixture of the foregoing wherein the stabilizer is selected from the group consisting of glycine, alanine, valine, leucine, isoleucine, methionine, threonine, isovaline, serine, histidine, tryptophan, proline, hydroxyproline, arginine, ornithine, asparagine, citrulline, aspartic acid, cysteine, glutamic acid, glutamine, lysine, hydroxylysine, phenylalanine, trans-4-hydroxy-L-proline, tyrosine, and a mixture of any of the foregoing, in an amount which is effective to prevent settling, creaming, or flocculation of the formulation for a time sufficient to allow reproducible dosing of the drug after agitation of the formulation.

12. A metered dose inhaler containing a medicinal aerosol formulation, the formulation comprising:
(a) a drug in particulate form in a therapeutically effective amount;
(b) a non-CFC propellant wherein the propellant is selected from the group consisting of 1,1,1,2-tetrafluoroethane, 1,1,1,2,3,3,3-heptafluoropropane or a mixture thereof; and
(c) a suitable stabilizer selected from an amino acid, or a mixture on the foregoing wherein the stabilizer is selected from the group consisting of glycine, alanine, valine, leucine, isoleucine, methionine, threonine, isovaline, serine, histidine, tryptophan, proline, hydroxyproline, arginine, ornithine, asparagine, citrulline, aspartic acid, cysteine, glutamic acid, glutamine, lysine, hydroxylysine, phenylalanine, trans-4-hydroxy-L-proline, tyrosine, and a mixture of any of the foregoing, present in an amount sufficient to stabilize the formulation to prevent settling, creaming or flocculation for a time sufficient to allow reproducible dosing of the drug after agitation of the formulation.

13. The metered dose inhaler as defined in claim 12 wherein said stabilizer is present in an amount of 0.000002% by weight to about 20% by weight based on the weight of the medicinal aerosol formulation.

14. The metered dose inhaler as defined in claim 12 wherein the drug is selected from the group consisting of albuterol, atropine, beclomethasone, beclomethasone monopropionate, beclomethasone dipropionate, budesonide, cromolyn, epinephrine, ephedrine, fentanyl, flunisolide, formoterol, ipratropium bromide, isoproterenol, pirbuterol, prednisone, salineterol, amiloride, fluticasone, an ester of fluticasone,(-)4-amino-3,5-dichloro-α-[[[6(2-pyndinyl)ethoxy] hexyl] amino] methyl]benzene-methanol and pharmaceutically acceptable hydrates, salts and solvates of the foregoing.

15. The metered dose inhaler as defined in claim 12 wherein the medicinal aerosol formulation further comprises a cosolvent.

16. The metered dose inhaler as defined in claim 15 wherein said cosolvent comprises ethanol.

## Patentansprüche

1. Medizinische Aerosolformulierung, welche umfasst:
(a) eine therapeutisch wirksame Menge eines in Teilchenform vorliegenden Medikaments;
(b) ein nicht-CFC Treibmittel, wobei das Treibmittel aus der Gruppe ausgewählt wird, die aus 1,1,1,2-Tetrafluorethan, 1,1,1,2,3,3,3-Heptafluorpropan oder einer Mischung derselben besteht;
(c) einen Stabilisator, der aus einer Aminosäure oder aus einer Mischung derselben ausgewählt wird, wobei der Stabilisator aus der Gruppe ausgewählt wird, bestehend aus Glycin, Alanin, Valin, Leucin, Isoleucin, Methionin, Threonin, Isovalin, Phenylalanin, Tyrosin, Serin, Histidin, Tryptophan, Prolin, Hydroxyprolin, Arginin, Ornithin, Asparagin, Citrullin, Asparaginsäure, Cystein, Glutaminsäure, Glutamin, Lysin, Hydroxylysin, trans-4-Hydroxy-L-prolin, und einer beliebigen Mischung aus den vorstehenden Verbindungen.

2. Formulierung wie in Anspruch 1 definiert, wobei das Medikament aus der Gruppe ausgewählt wird, bestehend aus Albuterol, Atropin, Beclomethason, Beclomethason-monopropionat, Beclomethason-dipropionat, Budesonid, Cromolyn, Epinephrin, Ephedrin, Fentanyl, Flunisolid, Formoterol, Ipratropiumbromid, Isoproterenol, Pirbuterol, Prednison, Salmeterol, Amilorid, Fluticason, Fluticasonester, (-)-4-Amino-3,5-dichlor-α-[[[[6-(2-pyridinyl)ethoxy]hexyl]amino]methyl]-benzol-methanol, und pharmazeutisch akzeptablen Salzen, Estern, Hydraten und Solvaten der vorstehenden Verbindungen.

3. Formulierung wie in Anspruch 1 definiert, welche ferner ein Cosolvens enthält.

4. Formulierung wie in Anspruch 3 definiert, wobei das Cosolvens Ethanol umfasst.

5. Formulierung wie in Anspruch 1 definiert, wobei der Stabilisator in einer Menge vorhanden ist, die wirksam ist, das Absetzen, die Bildung einer Creme oder das Ausflocken der Formulierung für eine Zeit zu verhindern, die ausreicht, um eine reproduzierbare Dosierung des Arzneimittels nach dem Schütteln der Formulierung zu ermöglichen.

6. Formulierung wie in Anspruch 5 definiert, wobei der Stabilisator in einer Menge im Bereich von 0,000002 Gew.-% bis 20 Gew.-% vorhanden ist, bezogen auf das Gewicht der Formulierung.

7. Verfahren zur Herstellung einer medizinischen Aerosolformulierung nach Anspruch 1, welches umfasst:
(a) Kombinieren (i) des Medikaments in einer Menge, die ausreichend ist, um eine Mehrzahl von therapeutisch wirksamen Dosen bereitzustellen, (ii) des Treibmittels in einer Menge, die ausreichend ist, um eine Mehrzahl der therapeutisch wirksamen Dosen aus einem Aerosolbehälter zu treiben, und (iii) des Stabilisators in einer Menge, die wirksam ist, die Formulierung zu stabilisieren; und
(b) Dispergieren der Bestandteile (i), (ii) und (iii).

8. Das Verfahren wie in Anspruch 7 definiert, welches in Schritt (a) ferner das Kombinieren der medizinischen Aerosolformulierung mit einem Cosolvens und in Schritt (b) das Dispergieren der Bestandteile (i), (ii), (iii) mit dem Cosolvens umfasst.

9. Verwendung einer Zusammensetzung nach Anspruch 1 für die Herstellung einer medizinischen Aerosolformulierung zur Behandlung von Asthma, der chronisch obstruktiven Lungenkrankheit, allergischer Rhinitis, Rhinitis, Diabetes, Angina oder lokalen Infektionen.

10. Formulierung nach Anspruch 1, die sich in einem Aerosolbehälter befindet, der mit einem einstellbaren Dosierventil ausgestattet ist.

11. Verfahren zum Stabilisieren einer Suspension einer Aerosolformulierung, die umfasst: ein nicht-CFC Treibmittel, welches aus der Gruppe ausgewählt wird, die aus 1,1,1,2-Tetrafluorethan, 1,1,1,2,3,3,3-Heptafluorpropan oder einer Mischung derselben besteht; und ein in Teilchenform vorliegendes Arzneimittel, wobei das Verfahren umfasst:
Einarbeiten eines Stabilisators in die Formulierung, wobei der Stabilisator ausgewählt ist aus der Gruppe, bestehend aus einer geeigneten Aminosäure oder aus einer beliebigen Mischung von Aminosäuren, wobei der Stabilisator aus der Gruppe ausgewählt wird, bestehend aus Glycin, Alanin, Valin, Leucin, Isoleucin, Methionin, Threonin, Isovalin, Serin, Histidin, Tryptophan, Prolin, Hydroxyprolin, Arginin, Ornithin, Asparagin, Citrullin, Asparaginsäure, Cystein, Glutaminsäure, Glutamin, Lysin, Hydroxylysin, Phenylalanin, trans-4-Hydroxy-L-prolin, Tyrosin, und einer beliebigen Mischung aus den vorstehenden Verbindungen, in einer Menge, die wirksam ist, das Absetzen, die Bildung einer Creme oder das Ausflocken der Formulierung für eine Zeit zu verhindern, die ausreicht, um eine reproduzierbare Dosierung des Arzneimittels nach dem ScKütteln der Formulierung zu ermöglichen.

12. Inhalator für eine einstellbare Dosis (MDI), der eine medizinische Aerosolformulierung enthält, wobei die Formulierung umfasst:
(a) ein Arzneimittel in Teilchenform in einer therapeutisch wirksamen Menge;
(b) ein nicht-CFC Treibmittel, wobei das Treibmittel aus der Gruppe ausgewählt wird, die aus 1,1,1,2-Tetrafluorethan, 1,1,1,2,3,3,3-Heptafluorpropan oder einer Mischung derselben besteht; und
(c) einen geeigneten Stabilisator, ausgewählt aus einer Aminosäure oder einer Mischung von Aminosäuren, wobei der Stabilisator aus der Gruppe ausgewählt wird, bestehend aus Glycin, Alanin, Valin, Leucin, Isoleucin, Methionin, Threonin, Isovalin, Serin, Histidin, Tryptophan, Prolin, Hydroxyprolin, Arginin, Ornithin, Asparagin, Citrullin, Asparaginsäure, Cystein, Glutaminsäure, Glutamin, Lysin, Hydroxylysin, Phenylalanin, trans-4-Hydroxy-L-prolin, Tyrosin, und einer beliebigen Mischung aus den vorstehenden Verbindungen, vorhanden in einer Menge, die ausreicht, um die Formulierung zu stabilisieren, und ein Absetzen, die Bildung einer Creme oder das Ausflocken für eine Zeit zu verhindern, die ausreicht, um eine reproduzierbare Dosierung des Arzneimittels nach dem Schütteln der Formulierung zu ermöglichen.

13. Der Inhalator für eine einstellbare Dosis (MDI) wie in Anspruch 12 definiert, wobei der Stabilisator in einer Menge von 0,000002 Gew.-% bis 20 Gew.-% vorhanden ist, bezogen auf das Gewicht der medizinischen Aerosolformulierung.

14. Der Inhalator für eine einstellbare Dosis (MDI) wie in Anspruch 12 definiert, wobei das Arzneimittel aus der Gruppe ausgewählt wird, bestehend aus Albuterol, Atropin, Beclomethason, Beclomethason-monopropionat, Beclomethason-dipropionat, Budesonid, Cromolyn, Epinephrin, Ephedrin, Fentanyl, Flunisolid, Formoterol, Ipratropiumbromid, Isoproterenol, Pirbuterol, Prednison, Salmeterol, Amilorid, Fluticason, Fluticasonester, (-)-4-Amino-3,5-dichlor-α-[[[[6-(2-pyridinyl)ethoxy]hexyl]amino]methyl]benzol-methanol, und pharmazeutisch akzeptablen Salzen, Estern, Hydraten und Solvaten der vorstehenden Verbindungen.

15. Der Inhalator für eine einstellbare Dosis (MDI) wie in Anspruch 12 definiert, wobei die medizinische Aerosolformulierung ferner ein Cosolvens umfasst.

16. Der Inhalator für eine einstellbare Dosis (MDI) wie in Anspruch 15 definiert, wobei das Cosolvens Ethanol umfasst.

## Revendications

1. Formulation médicinale d'aérosol, qui est constituée par :
(a) une quantité thérapeutiquement efficace d'un médicament particulaire;
(b) un propulsif non CFC dans où ledit propulsif est choisi dans le groupe constitué par le 1,1,1,2-tétrafluoroéthane, le 1,1,1,2,3,3,3-heptafluoropropane ou un mélange de ceux-ci ; et
(c) un stabilisant choisi parmi un acide aminé ou un mélange de ce qui précède, où ledit stabilisant est choisi dans le groupe constitué par la glycine, l'alanine, la valine, la leucine, l'isoleucine, la méthionine, la thréonine, l'isovaline, la phénylalanine, la tyrosine, la sérine, l'histidine, le tryptophane, la proline, l'hydroxyproline, l'arginine, l'ornithine, l'asparagine, la citrulline, l'acide aspartique, la cystéine, l'acide glutamique, la glutamine, la lysine, l'hydroxylysine, la trans-4-hydroxy-L-proline, et un mélange de l'un quelconque de ceux-ci.

2. Formulation telle que définie dans la revendication 1, dans laquelle le médicament est choisi dans le groupe constitué par l'albutérol, l'atropine, le béclométhasone, le monopropionate de béclométhasone, le dipropionate de béclométhasone, le budésonide, le cromolyn, l'épinéphrine, l'éphédrine, le fentanyl, le flunisolide, le formotérol, le bromure d'ipratropium, l'isoprotérénol, le pirbutérol, la prednisone, le salmétérol, l'amiloride, le fluticasone, les esters de fluticasone, le (-)4-amino-3,5-dichloro-α-[[[6(2-pyridinyl)éthoxy]hexyl]amino]méthyl]benzène-méthanol et les sels, les esters, les hydrates et les solvates pharmaceutiquement acceptables de ceux-ci.

3. Formulation telle que définie dans la revendication 1 qui est constituée en outre par un cosolvant.

4. Formulation telle que définie dans la revendication 3 dans laquelle ledit cosolvant est constitué par l'éthanol.

5. Formulation telle que définie dans la revendication 1, dans laquelle ledit stabilisant est présent en une quantité efficace pour empêcher le dépôt, le crémage ou la floculation de la formulation pendant une durée suffisante afin de permettre le dosage reproductible du médicament après l'agitation de la formulation.

6. Formulation telle que définie dans la revendication 5 dans laquelle ledit stabilisant est présent en une quantité dans la plage de 0,000002 % en poids à 20 % en poids par rapport au poids de la formulation.

7. Procédé de préparation d'une formulation médicinale d'aérosol selon la revendication 1, qui consiste à:
(a) combiner (i) ledit médicament en une quantité suffisante pour fournir une pluralité de doses thérapeutiquement efficaces, (ii) ledit propulsif en une quantité suffisante pour propulser une pluralité desdites doses thérapeutiquement efficaces d'une bouteille aérosol; et (iii) ledit stabilisant en une quantité efficace pour stabiliser la formulation ; et
(b) disperser les composants (i), (ii) et (iii).

8. Procédé tel que défini dans la revendication 7 dans lequel la formulation médicinale d'aérosol comprend en outre le fait de combiner dans l'étape (a) un cosolvant et dans l'étape (b) des composants de dispersion (i), (ii) et (iii) avec ledit cosolvant.

9. Utilisation d'une composition selon la revendication 1 pour la préparation d'une formulation médicinale d'aérosol destinée au traitement de l'asthme, d'une bronchopneumopathie chronique obstructive, d'une rhinite allergique, d'une rhinite, d'un diabète, d'une angine ou d'une infection locale.

10. Formulation selon la revendication 1, dans une bouteille aérosol dotée d'une valve doseuse.

11. Procédé destiné à stabiliser une formulation d'aérosol en suspension comprenant un propulsif non CFC où le propulsif est choisi dans le groupe constitué par le 1,1,1,2-tétrafluoroéthane, le 1,1,1,2,3,3,3-heptafluoropropane ou un mélange de ceux-ci, et un médicament particulaire qui consiste à
incorporer dans la formulation un stabilisant choisi parmi le groupe consistant en un acide aminé adapté ou tout mélange de celui-ci, dans lequel le stabilisant est choisi dans le groupe constitué par la glycine, l'alanine, la valine, la leucine, l'isoleucine, la méthionine, la thréonine, l'isovaline, la sérine, l'histidine, le tryptophane, la proline, l'hydroxyproline, l'arginine, l'ornithine, l'asparagine, la citrulline, l'acide aspartique, la cystéine, l'acide glutamique, la glutamine, la lysine, l'hydroxylysine, la phénylalanine, la trans-4-hydroxy-L-proline, la tyrosine, et un mélange de l'un quelconque de ceux-ci, en une quantité qui est efficace pour empêcher le dépôt, le crémage ou la floculation de la formulation pendant une durée suffisante afin de permettre le dosage reproductible du médicament après l'agitation de la formulation.

12. Inhalateur-doseur contenant une formulation médicinale d'aérosol, la formulation étant constituée par :
(a) un médicament sous une forme particulaire en une quantité thérapeutiquement efficace ;
(b) un propulsif non CFC où le propulsif est choisi dans le groupe constitué par le 1,1,1,2-tétrafluoroéthane, le 1,1,1,2,3,3,3-heptafluoropropane ou un mélange de ceux-ci ; et
(c) un stabilisant adapté choisi parmi un acide aminé ou un mélange de celui-ci, où le stabilisant est choisi dans le groupe constitué par la glycine, l'alanine, la valine, la leucine, l'isoleucine, la méthionine, la thréonine, l'isovaline, la sérine, l'histidine, le tryptophane, la proline, l'hydroxyproline, l'arginine, l'ornithine, l'asparagine, la citrulline, l'acide aspartique, la cystéine, l'acide glutamique, la glutamine, la lysine, l'hydroxylysine, la phénylalanine, la trans-4-hydroxy-L-proline, la tyrosine, et un mélange de l'un quelconque de ceux-ci, présent en une quantité suffisante pour stabiliser la formulation afin d'empêcher le dépôt, le crémage ou la floculation pendant une durée suffisante pour permettre le dosage reproductible du médicament après l'agitation de la formulation.

13. Inhalateur-doseur tel que défini dans la revendication 12, dans lequel ledit stabilisant est présent en une quantité de 0,000002 % en poids à 20 % en poids par rapport au poids de la formulation médicinale d'aérosol.

14. Inhalateur-doseur tel que défini dans la revendication 12 dans lequel le médicament est choisi dans le groupe constitué par l'albutérol, l'atropine, le béclométhasone, le monopropionate de béclométhasone, le dipropionate de béclométhasone, le budésonide, le cromolyn, l'épinéphrine, l'éphédrine, le fentanyl, le flunisolide, le formotérol, le bromure d'ipratropium, l'isoprotérénol, le pirbutérol, la prednisone, le salmétérol, l'amiloride, le fluticasone, l'ester de fluticasone, le (-)4-amino-3,5-dichloro-α-[[[6(2-pyridinyl)éthoxy]hexyl]amino]méthyl]benzène-méthanol et les sels, les hydrates et les solvates pharmaceutiquement acceptables de ceux-ci.

15. Inhalateur-doseur tel que défini dans la revendication 12 dans lequel la formulation médicinale d'aérosol est constituée en outre par un cosolvant.

16. Inhalateur-doseur tel que défini dans la revendication 15 dans lequel ledit cosolvant est constitué par l'éthanol.
